# EUROPEAN PATENT SPECIFICATION

(11) **EP 4 465 973 B1**
(45) Date of publication and mention of the grant of the patent: **29.10.2025**
(21) Application number: 23703831.0
(22) Date of filing: 19.01.2023
(51) Int. Cl.: A61K 9/20, A61K 31/454

(54) **PHARMACEUTICAL COMPOSITION OF A CYP11A1 INHIBITOR**
PHARMAZEUTISCHE ZUSAMMENSETZUNG EINES CYP11A1-INHIBITORS
COMPOSITION PHARMACEUTIQUE D'UN INHIBITEUR DE CYP11A1

(30) Priority: 20.01.2022 FI 20225044
(43) Date of publication of application: 27.11.2024
(73) Proprietor: ORION CORPORATION, 02200 Espoo (FI)
(72) Inventor: SALMIA, Jukka, 02700 Kauniainen (FI); SINGHAL, Mayank, MacClesfield, SK10 2TY (GB); TURUNEN, Elina, 02101 Espoo (FI)
(74) Representative: Jones Day
(86) International application number: PCT/FI2023/050043
(87) International publication number: WO 2023/139312

(56) References cited:
- WO-A1-2018/115591
- WO-A1-2022/184978

## Description

### Technical field

The present invention relates to a pharmaceutical composition for oral administration, particularly in the form of a tablet, or a powder suitable to be filled into a capsule shell, comprising 2-(isoindolin-2-ylmethyl)-5-((1-(methylsulfonyl)-piperidin-4-yl)methoxy)-4H-pyran-4-one (I) or a pharmaceutically acceptable salt thereof as an active ingredient.

### Background of the invention

The compound 2-(isoindolin-2-ylmethyl)-5-((1-(methylsulfonyl)piperidin-4-yl)methoxy)-4H-pyran-4-one (I) and derivatives thereof have been disclosed in WO 2018/115591. Compound of formula (I) is a selective inhibitor of CYP11A1 enzyme and is useful in the treatment of hormonally regulated diseases such as cancer including prostate cancer and breast cancer.

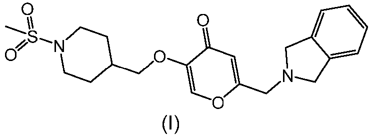

Compound (I) and pharmaceutically acceptable salts thereof have been found to be suitable for oral administration. However, when formulated together with carriers and excipients for the development of solid oral compositions, such as tablets and capsules, the active ingredient was found to be prone to stability issues. The suitable amount of the active ingredient in the solid oral compositions for the treatment of human patients was also found to be exceptionally small, generally from about 0.5 mg to about 10 mg, typically from about 1 mg to about 8 mg. Therefore, the percentage of the active ingredient in a tablet composition remains low as the tablet should not be too small making it difficult to handle by the patient.

### Summary of the invention

The present invention provides a pharmaceutical composition for oral administration, particularly in the form of a tablet, or a powder suitable to be filled into a capsule shell, comprising compound of formula (I) or a pharmaceutically acceptable salt thereof as an active ingredient. It has been found that the use of wet granulation, high intragranular drug load, neutral or slightly acidic pharmaceutical ingredients and/or inclusion of oxygen scavengers provide improved stability of the active ingredient. Moreover, it was found that the amount of the active ingredient sufficient in the composition for the treatment of human patients is exceptionally small. The composition provides good dose content uniformity and efficient release of the active ingredient and constant dissolution properties producing effective and reproducible in vivo plasma concentrations. Therefore, the composition according to the invention is particularly suitable as a dosage form for the treatment of patients suffering from hormonally regulated diseases where CYP11A1 inhibition is desired, such as prostate cancer and breast cancer.

Thus, according to one embodiment, the present invention discloses a wet granulated pharmaceutical composition comprising 2-(isoindolin-2-ylmethyl)-5-((1-(methylsulfonyl)piperidin-4-yl)methoxy)-4H-pyran-4-one (I) or a pharmaceutically acceptable salt thereof and at least one excipient.

According to another embodiment, the present invention provides a pharmaceutical composition comprising
(a) an intragranular part comprising at least 5 %, per weight of the intragranular part, of 2-(isoindolin-2-ylmethyl)-5-((1-(methylsulfonyl)piperidin-4-yl)-methoxy)-4H-pyran-4-one (I) or a pharmaceutically acceptable salt thereof, and at least one excipient; and
(b) an extragranular part comprising at least one excipient.

According to still another embodiment, the present invention provides a package comprising a pharmaceutical composition comprising 2-(isoindolin-2-yl-methyl)-5-((1-(methylsulfonyl)piperidin-4-yl)methoxy)-4H-pyran-4-one (I) or a pharmaceutically acceptable salt thereof and at least one excipient, the package also comprising an oxygen scavenger.

### Detailed description of the invention

The present invention relates to a pharmaceutical composition for oral administration, particularly in the form of a tablet, or a powder suitable to be filled into a capsule, comprising 2-(isoindolin-2-ylmethyl)-5-((1-(methylsulfonyl)-piperidin-4-yl)methoxy)-4H-pyran-4-one (I) or a pharmaceutically acceptable salt thereof as an active ingredient. Compound of formula (I) or a pharmaceutically acceptable salt thereof may be in amorphous or crystalline state. Suitable pharmaceutically acceptable salts of compound (I) are salts with organic or inorganic acids including salts with *p*-toluenesulfonic acid, 2-naphthalenesulfonic acid, 1,5-naphthalenedisulfonic acid, hydrobromic acid, nitric acid, benzenesulfonic acid, hydrochloric acid, maleic acid, 1,2-ethanedisulfonic acid, oxalic acid, ethanesulfonic acid, sulfuric acid and methanesulfonic acid. One particular embodiment of the invention is salts with *p*-toluenesulfonic acid, 2-naphthalenesulfonic acid, 1,5-naphthalenedisulfonic acid and hydrobromic acid. Another particular embodiment of the invention is a salt with *p*-toluenesulfonic acid.

In accordance with one embodiment of the present invention, there is provided a wet granulated pharmaceutical composition comprising 2-(isoindolin-2-ylmethyl)-5-((1-(methylsulfonyl)piperidin-4-yl)methoxy)-4H-pyran-4-one (I) or a pharmaceutically acceptable salt thereof and at least one excipient. Wet granulation has been found to protect compound (I) or a pharmaceutically acceptable salt thereof from degradation during manufacture and/or storage of the dosage form, such as tablets and powders, and tablets in particular.

According to another embodiment, the present invention provides a pharmaceutical composition comprising
(a) an intragranular part comprising at least 5 %, per weight of the intragranular part, of 2-(isoindolin-2-ylmethyl)-5-((1-(methylsulfonyl)piperidin-4-yl)-methoxy)-4H-pyran-4-one (I) or a pharmaceutically acceptable salt thereof, and at least one excipient; and
(b) an extragranular part comprising at least one excipient.

According to another embodiment, the present invention provides a package comprising a pharmaceutical composition comprising 2-(isoindolin-2-ylmethyl)-5-((1-(methylsulfonyl)piperidin-4-yl)methoxy)-4H-pyran-4-one (I) or a pharmaceutically acceptable salt thereof and at least one excipient, the package also comprising an oxygen scavenger. An oxygen scavenger has been found to improve the stability of the dosage form by preventing or inhibiting degradation of the active ingredient during the storage of the dosage form.

In a subclass of any of the above embodiments are compositions comprising an intragranular part which comprises at least about 5 %, for example from about 5 % to about 20 %, from about 5 % to about 15 %, or from about 5 % to about 12 %, per weight of the intragranular part, of 2-(isoindolin-2-ylmethyl)-5-((1-(methylsulfonyl)piperidin-4-yl)methoxy)-4H-pyran-4-one (I) or a pharmaceutically acceptable salt thereof, and at least one excipient.

In a subclass of the above embodiment are pharmaceutical compositions which comprise
(a) an intragranular part comprising at least 5 %, for example from about 5 % to about 20 %, from about 5 % to about 15 %, or from about 5 % to about 12 %, per weight of the intragranular part, of 2-(isoindolin-2-ylmethyl)-5-((1-(methylsulfonyl)-piperidin-4-yl)methoxy)-4H-pyran-4-one (I) or a pharmaceutically acceptable salt thereof, and at least one excipient; and
(b) an extragranular part comprising at least one excipient.

In a subclass of any of the above embodiments are pharmaceutical compositions comprising from about 0.5 mg to about 10 mg, for example from about 1 mg to about 8 mg, for example about 2.5 mg, about 3.5 mg, about 5 mg or about 7.1 mg, of 2-(isoindolin-2-ylmethyl)-5-((1-(methylsulfonyl)piperidin-4-yl)methoxy)-4H-pyran-4-one (I) or a pharmaceutically acceptable salt thereof.

High drug load in the intragranular part has been found to improve the stability of compound of formula (I) or a pharmaceutically acceptable salt thereof. Thus, even if the suitable amount of compound of formula (I) or a pharmaceutically acceptable salt thereof in the dosage form is small, it is preferred to maintain high drug load in the intragranular part in order to improve stability of the dosage form during manufacture and/or storage.

In a subclass of any of the above embodiments are pharmaceutical compositions comprising
(a) an intragranular part comprising, per weight of the intragranular part,
   (i) at least about 5 %, for example from about 5 % to about 20 %, from about 5 % to about 15 % or from about 5 % to about 12 %, of 2-(isoindolin-2-ylmethyl)-5-((1-(methylsulfonyl)piperidin-4-yl)methoxy)-4H-pyran-4-one (I) or a pharmaceutically acceptable salt thereof;
   (ii) from about 55 % to about 95 %, for example from about 70 to about 90 %, of a filler;
   (iii) from about 0.5 % to about 10 %, for example from about 1 to about 5 %, of a disintegrant; and
   (iv) from about 1 % to about 15 %, for example from about 2 to about 10 %, of a binder; and
(b) an extragranular part comprising, per weight of the extragranular part,
   (i) from about 50 % to 100 %, for example from about 70 to about 100 %, of a filler;
   (ii) from 0 % to about 25 %, for example from about 0 to about 20 %, or from about 2 to about 15 %, of a disintegrant; and
   (iii) from 0 % to about 25 %, for example from about 0 to about 20 %, or from about 2 to about 15 %, of a lubricant.

As used herein, a "filler" refers to one or more pharmaceutically acceptable excipient(s) that adds bulkiness to a pharmaceutical composition. Examples of fillers include sugars (e.g., mannitol or sucrose), starch, pregelatinized starch, microcrystalline cellulose, lactose, calcium hydrogen phosphate and sorbitol. According to one embodiment, the filler is selected from mannitol, pregelatinized starch, microcrystalline cellulose, lactose, calcium hydrogen phosphate and a combination thereof. According to another embodiment, the filler is selected from mannitol, pregelatinized starch, microcrystalline cellulose, and a combinations thereof.

The term "calcium hydrogen phosphate" or "dicalcium phosphate", as used herein, includes anhydrous calcium hydrogen phosphate and hydrates such as calcium hydrogen phosphate dihydrate.

As used herein, a "disintegrant" refers to one or more pharmaceutically acceptable excipient(s) which is added to the pharmaceutical composition to cause its disintegration to support the release of the active ingredient from the pharmaceutical composition. Examples of disintegrants include croscarmellose sodium, cross-linked polyvinylpyrrolidone and sodium starch glycolate. According to one embodiment, the disintegrant is selected from croscarmellose sodium, sodium starch glycolate and a combination thereof. According to another embodiment, the disintegrant is croscarmellose sodium.

As used herein, a "binder" refers to one or more pharmaceutically acceptable excipient(s) that imparts enhanced cohesion by binding the active ingredient and the excipients together in a mixture. Examples of binders include hydroxypropylmethyl cellulose (HPMC), hydroxypropyl cellulose (HPC), polyvinylpyrrolidone (PVP), polyvinyl acetate and polyvinyl alcohol. According to one embodiment, the binder is selected from hydroxypropylmethyl cellulose (HPMC), hydroxypropyl cellulose (HPC) and a combination thereof. According to another preferred embodiment, the binder is hydroxypropylmethyl cellulose (HPMC). According to still another embodiment, the binder is hydroxypropylmethyl cellulose (HPMC) and the disintegrant is croscarmellose sodium.

As used herein, a "lubricant" refers to one or more pharmaceutically acceptable excipient(s), which is added to the pharmaceutical composition to reduce friction, heat, and wear when introduced between solid surfaces. Examples of lubricants include sodium stearyl fumarate, magnesium stearate, stearic acid, talc, silica, calcium stearate and carnauba wax. According to one embodiment, the lubricant is sodium stearyl fumarate.

In a subclass of any of the above embodiments are pharmaceutical compositions comprising
(a) an intragranular part comprising, per weight of the intragranular part,
   (i) at least about 5 %, for example from about 5 % to about 20 %, from about 5 % to about 15 % or from about 5 % to about 12 %, of 2-(isoindolin-2-ylmethyl)-5-((1-(methylsulfonyl)piperidin-4-yl)methoxy)-4H-pyran-4-one (I) or a pharmaceutically acceptable salt thereof;
   (ii) from about 55 % to about 95 %, for example from about 70 % to about 90 %, of a filler selected from mannitol, pregelatinized starch, microcrystalline cellulose, lactose, calcium hydrogen phosphate and a combination thereof;
   (iii) from about 0.5 % to about 10 %, for example from about 1 % to about 5 %, of a disintegrant selected from croscarmellose sodium, sodium starch glycolate and a combination thereof; and
   (iv) from about 1 % to about 15 %, for example from about 2 % to about 10 %, of a binder selected from HPMC, HPC and a combination thereof; and
(b) an extragranular part comprising, per weight of the extragranular part,
   (i) from about 50 % to 100 %, for example from about 70 % to about 100 %, of a filler selected from microcrystalline cellulose, mannitol, lactose, calcium hydrogen phosphate and a combination thereof;
   (ii) from 0 % to about 25 %, for example from about 0 % to about 20 %, or from about 2 to about 15 %, of a disintegrant selected from croscarmellose sodium, sodium starch glycolate and a combination thereof; and
   (iii) from 0 % to about 25 %, for example from about 0 % to about 20 %, or from about 2 to about 15 %, of a lubricant wherein the lubricant is sodium stearyl fumarate.

In a subclass of any of the above embodiments are pharmaceutical compositions comprising
(a) an intragranular part comprising, per weight of the intragranular part,
   (i) at least about 5 %, for example from about 5 % to about 20 %, from about 5 % to about 15 % or from about 5 % to about 12 %, of 2-(isoindolin-2-ylmethyl)-5-((1-(methylsulfonyl)piperidin-4-yl)methoxy)-4H-pyran-4-one (I) or a pharmaceutically acceptable salt thereof;
   (ii) from about 55 % to about 95 %, for example from about 70 % to about 90 %, of a combination of mannitol and pregelatinized starch;
   (iii) from about 0.5 % to about 10 %, for example from about 1 % to about 5 %, of croscarmellose sodium;
   (iv) from about 1 % to about 15 %, for example from about 2 % to about 10 %, of HPMC; and
(b) an extragranular part comprising, per weight of the extragranular part,
   (i) from about 50 % to 100 %, for example from about 70 % to about 90 %, of microcrystalline cellulose;
   (ii) from 0 % to about 25 %, for example from about 2 % to about 20 %, or from about 5 to about 15 %, of croscarmellose sodium; and
   (iii) from 0 % to about 25 %, for example from about 2 % to about 20 %, or from about 5 to about 15 %, of sodium stearyl fumarate.

In a subclass of any of the above embodiments are pharmaceutical compositions, wherein the intragranular part comprises, from about 30 % to about 95 %, for example from about 50 % to about 90 % or from about 70 % to about 85 %, per weight of the composition.

It has been found that fine particles of the compound of formula (I) or a pharmaceutically acceptable salt thereof provide better dissolution rate and content uniformity than coarse particles.

Thus, for the manufacture of the pharmaceutical composition, compound of formula (I) or a pharmaceutically acceptable salt thereof, for example p-toluenesulfonic acid salt, is suitably milled. In various embodiments, the milled compound has a particle size having the volume median diameter (Dv50) of not more than 50 µm, not more than 30 µm, or not more than 25 µm. For example, Dv50 may be in the range of 1 - 25 µm, between 2 - 20 µm, or between 5 - 15 µm. The particle size distribution can be analyzed by laser light diffraction, for example using Beckman Coulter LS13320 laser diffraction particle size analyzer equipped with Tornado Dry Powder System using air as dispersion medium with measurement pressure 24"H₂O + 2"H₂O, sample amount 10 ml, system controlled target 5 % for obscuration and applying Fraunhofer optical model.

Milling of the active ingredient can be carried out using suitable feeder and milling equipment, for example, single or twin screw/auger feeders, hammer mills, pin mills, jet mills or sieve mills, using suitable rotor speeds such as, for example 3000 - 10000 rpm. The milling can be conducted in a suitable temperature, for example, in room temperature or lower.

Thus, in a further subclass of any of the above embodiments is a pharmaceutical composition wherein the composition provides a dissolution of at least 80 % per weight of 2-(isoindolin-2-ylmethyl)-5-((1-(methylsulfonyl)piperidin-4-yl)methoxy)-4H-pyran-4-one (I) or a pharmaceutically acceptable salt thereof within 60 minutes, preferably within 45 minutes, in 0.05 M phosphate buffer pH 6.8 using paddle apparatus (USP apparatus 2) with paddle speed of 75 rpm at 37 °C and vessel volume of 500 ml. In one embodiment, the active ingredient is a p-toluenesulfonic acid salt of compound (I) wherein the pharmaceutical composition provides dissolution of at least 80 % per weight of *p*-toluenesulfonic acid salt of 2-(isoindolin-2-ylmethyl)-5-((1-(methylsulfonyl)piperidin-4-yl)methoxy)-4H-pyran-4-one (I) within 60 minutes, or within 45 minutes, in 0.05 M phosphate buffer pH 6.8 using paddle apparatus (USP apparatus 2) with paddle speed of 75 rpm at 37 °C and vessel volume of 500 ml.

In a further subclass of any of the above embodiments is a pharmaceutical composition which is in the form of a coated or uncoated tablet. According to another subclass of any of the above embodiments, the pharmaceutical composition is in the form of a powder, including granules, which may be suitable to be filled into a capsule shell.

Thus, the term "powder", as used herein, refers to powdery material such as milled powder or granules.

In a further subclass of any of the above embodiments is a pharmaceutical composition which is prepared by a method comprising wet granulation.

It is to be understood that when the composition is in the form of a powder, including granules, the component percentages above refer to the powder pharmaceutical composition without the capsule shell.

The compositions are suitably prepared by wet granulation as wet granulation has been found to protect compound of formula (I) or a pharmaceutically acceptable salt thereof from degradation during manufacture and/or storage of the dosage form.

Thus, in accordance with one embodiment of the invention the process for manufacturing a pharmaceutical composition according to any of the above embodiments is characterized by the steps of (a) mixing compound of formula (I) or a pharmaceutically acceptable salt thereof and other intragranular components in a suitable mixer; (b) granulating the mixture by spraying granulating liquid, for example water, into the mixture; (c) drying the wet granules; (d) mixing the extragranular components with the dried granules; and, in case the composition is a tablet, (e) compressing the obtained mixture into tablets. In case the composition is a powder, the mixture of step (d) can be filled directly into a capsule shell.

In accordance with another embodiment of the invention the process for manufacturing a pharmaceutical composition according to any of the above embodiment is characterized by the steps of (a) mixing compound of formula (I) or a pharmaceutically acceptable salt thereof and other intragranular components except at least part of the binder in a suitable mixer; (b) granulating the mixture by spraying combination of granulating liquid, for example water, and at least part of the binder into the mixture; (c) drying the wet granules; (d) mixing the extragranular components with the dried granules; and, in case the composition is a tablet, (e) compressing the obtained mixture into tablets. In case the composition is a powder, the mixture of step (d) can be filled directly into a capsule shell.

According to another embodiment, all components are blended together and filled into a capsule shell without granulation step.

According to one embodiment, the composition of the invention is suitably manufactured, for example, by first mixing compound of formula (I) or a pharmaceutically acceptable salt thereof, filler, binder and at least part of the disintegrant (typically from about 30 % to about 70 %, for example about 50 %, per weight, of the total) in a suitable granulator vessel, for example wet high shear granulator. The mixture is then suitably granulated in the granulator using purified water as granulating liquid. Alternatively, the binder can be mixed with the granulation liquid instead of mixing the binder with other intragranular components in the first step. The wet granules can then be screened, for example, using a screening mill unit (rotating impeller) and subsequently dried, for example, in a fluid bed dryer.

The dried granules may then be screened with a screening apparatus, for example a screening mill. When the composition is a powder to be filled into a capsule shell, the extragranular filler and optional other ingredients are mixed with the granules. The obtained powder can then be filled into a capsule shell, for example a gelatin capsule shell or a HPMC capsule shell. When the composition is a tablet, the extragranular filler and the possible rest of the disintegrant may be added to the granules followed by blending the mixture in, for example, a diffusion mixer. The lubricant is added to the mass of the previous step followed by blending. The tablet mass is then compressed into tablet cores, for example, in a power assisted rotary tablet press.

The tablet cores can be provided with a water-soluble film coating, if desired, to facilitate tablet swallowing, to protect from direct contact with the drug substance and to improve aesthetics. A suitable film coating agent may be selected from the group consisting of plasticizers, film-forming agents, surfactants, colorants and flavouring agents. Optionally an anti-tacking agent, glidant or opacifier may be used. A plasticizer, such as polyethylene glycol (PEG) or glycerol, a film-forming agent such as hydroxypropylmethyl cellulose (HPMC), a colorant, such as ferric oxide or titanium dioxide, a glidant such as talc or magnesium stearate, and a flavouring agent such as polydextrose or sucrose, may be combined with a film-coating liquid, preferably water, to result in a homogeneous coating suspension which is brought up, preferably sprayed, on the tablets in a suitable coating device, such as for example a perforated drum coater. The film coating typically amounts to from about 2 % to about 5 %, for example from about 2.5 % to about 4 %, per weight of the tablet composition.

The unit dose tablet or unit dose powder to be filled into a capsule shell typically contains from about 0.5 mg to about 10 mg, for example from about 1 mg to about 8 mg, for example about 2.5 mg, about 3.5 mg, about 5.0 mg or about 7.1 mg, of 2-(isoindolin-2-ylmethyl)-5-((1-(methylsulfonyl)piperidin-4-yl)methoxy)-4H-pyran-4-one (I) or a pharmaceutically acceptable salt thereof, for example, a salt of p-toluenesulfonic acid. The unit dose tablet or unit dose powder typically has the weight from about 50 mg to about 100 mg, for example from about 70 mg to about 90 mg. The tablet may have, for example, oval or round shape and have a diameter typically from about 4 mm to about 8 mm, for example from about 5 mm to about 7 mm, for example 6 mm.

The tablets or capsules can be packaged in conventional pharmaceutical containers such as bottles or blisters, for example HDPE bottles or aluminium foiled PET blister packs. An oxygen scavenger has been found to improve the stability of the dosage form by preventing or inhibiting degradation of the active ingredient during storage of the dosage form. Therefore, the container may contain an oxygen scavenger. The term "oxygen scavenger" refers to material which consume, deplete or reduce the amount of oxygen from a given environment. Examples of oxygen scavenger materials comprise transition metals or salts thereof including, but not limited to, manganese II or III, iron II or III, cobalt II or III, nickel II or III, copper I or II, rhodium II, III or IV, and ruthenium, and non-metallic materials such as ascorbic acid and salts thereof, ethylene glycol, propylene glycol, glyceric acid, gallic acid, catechol, unsaturated hydrocarbons and hydrogenated rubbers. Small canisters containing oxygen scavenger material are commercially available, for example PharmaKeep^{®} CD Canisters from Airnov Healthcare Packaging and StabilOx^{®} Canisters from Multisorb Filtration Group. Such canister can be included in the pharmaceutical container such as plastic bottle together with tablets or capsules.

Compound of formula (I) or a pharmaceutically acceptable salt thereof is suitably administered, for example for the treatment of hormone dependent cancers, for example prostate cancer, in an amount ranging from about 0.5 mg to about 30 mg, or from about 1 mg to about 25 mg, for example from about 2 mg to about 15 mg, such as about 5 mg or about 7 mg per day to the patient. The dose can be administered once daily or divided to several times a day, for example twice daily.

The invention is further illustrated by the following examples.

### Example 1. Tablet composition

| **Composition** | **Amount (mg)** |
|---|---|
| **Intragranular** | |
| Compound (I), *p*-toluenesulfonic acid salt | 3.5 |
| Mannitol | 43.5 |
| Pregelatinized starch | 9.7 |
| Hydroxypropyl methylcellulose (HPMC) | 2.8 |
| Croscarmellose sodium | 1.4 |

| **Extragranular** | |
|---|---|
| Microcrystalline cellulose | 11.3 |
| Croscarmellose sodium | 1.4 |
| Sodium stearyl fumarate | 1.4 |
| **Weight (uncoated tablet)** | 75.0 |
| **Film coating** ^{a)} | 3.0 |
| **Weight (coated tablet)** | 78.0 |

| | |
|---|---|
| a) Contains HPMC, TiO₂, talc, polydextrose and PEG 3350 | |

### Example 2. Tablet composition

| **Composition** | **Amount (mg)** |
|---|---|
| **Intragranular** | |
| Compound (I), *p*-toluenesulfonic acid salt | 7.1 |
| Mannitol | 40.5 |
| Pregelatinized starch | 9.1 |
| Hydroxypropyl methylcellulose (HPMC) | 2.8 |
| Croscarmellose sodium | 1.4 |

| **Extragranular** | |
|---|---|
| Microcrystalline cellulose | 11.3 |
| Croscarmellose sodium | 1.4 |
| Sodium stearyl fumarate | 1.4 |
| **Weight (uncoated tablet)** | 75.0 |
| **Film coating** ^{a)} | 3.0 |
| **Weight (coated tablet)** | 78.0 |

| | |
|---|---|
| a) Contains HPMC, TiO₂, talc, polydextrose and PEG 3350 | |

### Example 3. Tablet composition

| **Composition** | **Amount (mg)** |
|---|---|
| **Intragranular** | |
| Compound (I), *p*-toluenesulfonic acid salt | 7.1 |
| Mannitol | 46.7 |
| Pregelatinized starch | 10.4 |
| Hydroxypropyl methylcellulose (HPMC) | 3.2 |
| Croscarmellose sodium | 1.6 |

| **Extragranular** | |
|---|---|
| Microcrystalline cellulose | 12.8 |
| Croscarmellose sodium | 1.6 |
| Sodium stearyl fumarate | 1.6 |
| **Weight (uncoated tablet)** | 85.0 |
| **Film coating ^{a)}** | 2.6 |
| **Weight (coated tablet)** | 87.6 |

| | |
|---|---|
| a) Contains HPMC, TiO₂, sucrose, magnesium stearate, polysorbate 80 and glycerol | |

The tablet compositions of Examples 1 to 3 are prepared by mixing the intragranular components in a high-shear mixer. The mixture is granulated by spraying water into the mixture. The granules are dried in a fluid-bed dryer and screened with a conical mill. Extragranular components are added, and the blend is compressed into tablets with a tablet press. Finally, the tablets are coated by spraying the coating suspension on the tablet cores in a heated pan coater until the theoretical weight gain in tablets is reached.

### Example 4. Tablet composition

| **Composition** | **Amount (mg)** |
|---|---|
| **Intragranular** | |
| Compound (I), *p*-toluenesulfonic acid salt | 3.5 |
| Lactose | 42.0 |
| Pregelatinized starch | 9.3 |
| Hydroxypropyl methylcellulose (HPMC) | 2.8 |
| Croscarmellose sodium | 1.4 |

| **Extragranular** | |
|---|---|
| Microcrystalline cellulose | 23.1 |
| Croscarmellose sodium | 1.4 |
| Sodium stearyl fumarate | 1.4 |
| **Total weight** | 85.0 |

### Example 5. Tablet composition

| **Composition** | **Amount (mg)** |
|---|---|
| **Intragranular** | |
| Compound (I), *p*-toluenesulfonic acid salt | 3.5 |
| Mannitol | 43.3 |
| Microcrystalline cellulose | 10.9 |
| Hydroxypropyl methylcellulose (HPMC) | 2.8 |
| Croscarmellose sodium | 1.4 |

| **Extragranular** | |
|---|---|
| Microcrystalline cellulose | 12.3 |
| Croscarmellose sodium | 2.4 |
| Sodium stearyl fumarate | 1.4 |
| **Total weight** | 78.0 |

### Example 6. Tablet composition

| **Composition** | **Amount (mg)** |
|---|---|
| **Intragranular** | |
| Compound (I), *p*-toluenesulfonic acid salt | 3.5 |
| Mannitol | 42.0 |
| Pregelatinized starch | 10.0 |
| Hydroxypropyl methylcellulose (HPMC) | 3.0 |
| Croscarmellose sodium | 2.0 |

| **Extragranular** | |
|---|---|
| Microcrystalline cellulose | 15.5 |
| Croscarmellose sodium | 2.0 |
| Sodium stearyl fumarate | 2.0 |
| **Total weight** | 80.0 |

### Example 7. Tablet composition

| **Composition** | **Amount (mg)** |
|---|---|
| **Intragranular** | |
| Compound (I), *p*-toluenesulfonic acid salt | 7.1 |
| Mannitol | 40.5 |
| Pregelatinized starch | 9.1 |
| Hydroxypropyl methylcellulose (HPMC) | 2.8 |
| Sodium starch glycolate | 1.4 |

| **Extragranular** | |
|---|---|
| Mannitol | 11.3 |
| Sodium starch glycolate | 1.4 |
| Sodium stearyl fumarate | 1.4 |
| **Total weight** | 75.0 |

### Example 8. Tablet composition

| **Composition** | **Amount (mg)** |
|---|---|
| **Intragranular** | |
| Compound (I), *p*-toluenesulfonic acid salt | 7.1 |
| Mannitol | 48.0 |
| Pregelatinized starch | 8.1 |
| Hydroxypropyl methylcellulose (HPMC) | 3.2 |
| Croscarmellose sodium | 1.6 |

| **Extragranular** | |
|---|---|
| Microcrystalline cellulose | 6.4 |
| Mannitol | 6.4 |
| Croscarmellose sodium | 1.6 |
| Sodium stearyl fumarate | 1.6 |
| **Total weight** | 84.0 |

### Example 9. Tablet composition

| **Composition** | **Amount (mg)** |
|---|---|
| **Intragranular** | |
| Compound (I), *p*-toluenesulfonic acid salt | 3.5 |
| Mannitol | 41.4 |
| Microcrystalline cellulose | 9.2 |
| Hydroxypropyl cellulose (HPC) | 3.5 |
| Croscarmellose sodium | 2.4 |

| **Extragranular** | |
|---|---|
| Microcrystalline cellulose | 15.1 |
| Croscarmellose sodium | 2.4 |
| Sodium stearyl fumarate | 2.5 |
| **Total weight** | 80.0 |

### Example 10. Tablet composition

| **Composition** | **Amount (mg)** |
|---|---|
| **Intragranular** | |
| Compound (I), *p*-toluenesulfonic acid salt | 3.5 |
| Mannitol | 42.0 |
| Pregelatinized starch | 10.0 |
| Hydroxypropyl cellulose (HPC) | 3.5 |
| Sodium starch glycolate | 2.4 |

| **Extragranular** | |
|---|---|
| Microcrystalline cellulose | 13.7 |
| Sodium starch glycolate | 2.4 |
| Sodium stearyl fumarate | 2.5 |
| **Total weight** | 80.0 |

### Example 11. Tablet composition

| **Composition** | **Amount (mg)** |
|---|---|
| **Intragranular** | |
| Compound (I), *p*-toluenesulfonic acid salt | 7.1 |
| Mannitol | 40.3 |
| Pregelatinized starch | 10.4 |
| Hydroxypropyl methylcellulose (HPMC) | 3.2 |
| Croscarmellose sodium | 1.6 |
| Calcium hydrogen phosphate, anhydrous | 6.4 |

| **Extragranular** | |
|---|---|
| Microcrystalline cellulose | 12.8 |
| Croscarmellose sodium | 2.4 |
| Sodium stearyl fumarate | 2.4 |
| **Total weight** | 86.6 |

### Example 12. Tablet composition

| **Composition** | **Amount (mg)** |
|---|---|
| **Intragranular** | |
| Compound (I), *p*-toluenesulfonic acid salt | 1.4 |
| Mannitol | 17.4 |
| Pregelatinized starch | 3.9 |
| Croscarmellose sodium | 0.6 |
| Hydroxypropyl cellulose (HPC) | 1.1 |

| **Extragranular** | |
|---|---|
| Microcrystalline cellulose | 47.8 |
| Croscarmellose sodium | 1.4 |
| Sodium stearyl fumarate | 1.4 |
| **Total weight** | 75.0 |

The tablet compositions of Examples 4 to 12 are prepared by mixing the intragranular components in a high-shear mixer. The mixture is granulated by spraying water into the mixture. The granules are dried in a fluid-bed dryer and screened with a conical mill. Extragranular components are added, and the blend is compressed into tablets with a tablet press.

### Example 13. Powder composition to be filled into a capsule shell

| **Composition** | **Amount (mg)** |
|---|---|
| **Intragranular** | |
| Compound (I), *p*-toluenesulfonic acid salt | 3.5 |
| Mannitol | 43.5 |
| Pregelatinized starch | 9.7 |
| Hydroxypropyl methylcellulose (HPMC) | 2.8 |
| Croscarmellose sodium | 1.4 |

| **Extragranular** | |
|---|---|
| Microcrystalline cellulose | 14.1 |
| **Total weight** | 75.0 |

### Example 14. Powder composition to be filled into a capsule shell

| **Composition** | **Amount (mg)** |
|---|---|
| **Intragranular** | |
| Compound (I), toluenesulfonic acid salt | 7.1 |
| Mannitol | 40.5 |
| Pregelatinized starch | 9.1 |
| Hydroxypropyl methylcellulose (HPMC) | 2.8 |
| Croscarmellose sodium | 1.4 |

| **Extragranular** | |
|---|---|
| Microcrystalline cellulose | 14.1 |
| **Total weight** | 75.0 |

The powder compositions of Examples 13 and 14 are prepared by mixing the intragranular components in a high-shear mixer. The mixture is granulated by spraying water into the mixture. The granules are dried in a fluid-bed dryer and screened with a conical mill. Extragranular components are added followed by blending. The blend is filled into capsule shells.

### Example 15. Comparative stability study I

Various wet granulated (WG) tablet compositions WG1 to WG9 were prepared using the method as described for Examples 4-12. The stability of the compositions was tested by storing the tablets in 60 °C / 75 % RH conditions for 4 weeks. The formulation descriptions and total amount of formed impurities after 4 weeks are given in the Table 1. The quantitative compositions of the formulations are presented in Table 2. The formulations WG6 to WG8 did not contain any extragranular components and suffered from poor tablet quality. It can be seen that croscarmellose sodium appears to improve stability in HPMC containing compositions.

**Table 1. Results of the comparative stability study**

| **Composition Description** | | **Total impurities** |
|---|---|---|
| WG1 | Composition of WG1 | 0.47 % |
| WG2 | WG1 with DCP added | 0.54 % |
| WG3 | WG1 with mannitol changed to lactose | 0.49 % |
| WG4 | WG1 without CCS | 0.94 % |
| WG5 | WG1 without HPMC | 0.38 % |
| WG6 | API+ mannitol + starch | 0.24 % |
| WG7 | API+ mannitol + starch + MCC | 0.26 % |
| WG8 | API+ mannitol + starch + MCC + CCS | 0.41 % |
| WG9 | API+ mannitol + starch + MCC + CCS + SSF | 0.41 % |

| | | |
|---|---|---|
| API = Active pharmaceutical ingredient (Compound (I) p-toluenesulfonic acid salt) DCP = Dicalcium phosphate (calcium hydrogen phosphate), anhydrous CCS = Croscarmellose sodium MCC = Microcrystalline cellulose SSF = Sodium stearyl fumarate | | |

**Table 2. Quantitative formulation of compositions WG1 to WG9 (WG3 similar to WG1 except that mannitol is changed to lactose)**

| **Components (%)** | **WG1** | **WG2** | **WG4** | **WG5** | **WG6** | **WG7** | **WG8** | **WG9** |
|---|---|---|---|---|---|---|---|---|
| Intragranular | | | | | | | | |
| API | 8.31 | 8.31 | 8.63 | 8.63 | 11.00 | 9.17 | 8.31 | 8.31 |
| Mannitol | 54.95 | 47.45 | 57.10 | 57.10 | 72.75 | 60.66 | 54.95 | 53.07 |
| Pregelatinized starch | 12.27 | 12.27 | 12.75 | 12.75 | 16.25 | 13.55 | 12.27 | 12.27 |
| DCP | - | 7.50 | - | - | - | - | - | |
| HPMC | 3.77 | 3.77 | 3.91 | - | - | - | - | |
| MCC | - | - | - | - | - | 16.62 | 15.06 | 15.06 |
| CCS | 1.88 | 1.88 | - | 1.95 | - | - | 9.41 | 9.41 |

| Extragranular | | | | | | | | |
|---|---|---|---|---|---|---|---|---|
| MCC | 15.06 | 15.06 | 15.65 | 15.65 | - | - | - | - |
| CCS | 1.88 | 1.88 | - | 1.96 | - | - | - | - |
| SSF | 1.88 | 1.88 | 1.96 | 1.96 | - | - | - | 1.88 |
| **Total** | **100** | **100** | **100** | **100** | **100** | **100** | **100** | **100** |

| | | | | | | | | |
|---|---|---|---|---|---|---|---|---|
| API = Active pharmaceutical ingredient (Compound (I) toluenesulfonic acid salt) DCP = Dicalcium phosphate (calcium hydrogen phosphate), anhydrous CCS = Croscarmellose sodium MCC = Microcrystalline cellulose SSF = Sodium stearyl fumarate | | | | | | | | |

### Example 16. Comparative stability study II

The stability of a wet granulated (WG) tablet according to Example 3 (without film coating) and a corresponding direct compressed (DC) tablet having an equivalent quantitative composition was studied. Other direct compressed tablets were also prepared for the study. The stability of the compositions was tested by storing the tablets in 60 °C / 75 % RH conditions for 4 weeks. The formulation descriptions and total amount of formed impurities after 4 weeks are given in the Table 3. The actual compositions of direct compressed formulations DC1 to DC4 are presented in Table 4. It can be seen that wet granulated composition has better stability than corresponding direct compressed composition.

**Table 3. Results of the comparative stability study**

| **Composition Description** | | **Total impurities** |
|---|---|---|
| WG10 | Composition of Example 3 without film coating (Ex. 3) | 0.6 % |
| DC1 | Direct compressed otherwise equivalent to Ex. 3 | 1.3 % |
| DC2 | Direct compressed Ex. 3 like without HPMC | 1.8 % |
| DC3 | Direct compressed Ex. 3 like without HPMC and CCS | 2.0 % |
| DC4 | Direct compressed Ex. 3 like without MCC, HPMC and CCS | 3.4 % |

| | | |
|---|---|---|
| MCC = microcrystalline cellulose CCS = Croscarmellose sodium | | |

**Table 4. Quantitative formulation of direct compressed (DC) compositions**

| **Components (%)** | **DC1** | **DC2** | **DC3** | **DC4** |
|---|---|---|---|---|
| Compound (I), toluenesulfonic acid salt | 8.3 | 8.3 | 8.3 | 8.3 |
| Mannitol | 54.9 | 55.8 | 59.0 | 76.1 |
| Pregelatinized starch | 12.3 | 9.6 | 10.2 | 13.1 |
| Microcrystalline cellulose | 15.0 | 20.0 | 20.0 | - |
| HPMC | 3.8 | - | - | - |
| Croscarmellose sodium | 3.8 | 3.8 | - | - |
| Sodium stearyl fumarate | 1.9 | 2.5 | 2.5 | 2.5 |
| **Total** | **100** | **100** | **100** | **100** |

The direct compressed tablets were prepared by blending the components together and compressing the mixture into tablets with a tablet press.

### Example 17. Comparative stability study III

The stability of wet granulated (WG) tablets WG11 to WG15 was studied. The tablets were prepared using the method as described for Examples 4-12. The stability of the tablets was tested by storing 65 tablets in closed 60 ml or 100 ml HDPE bottle with 2 g of silica desiccant in 40 °C / 75 % RH conditions for 6 months. The formulation descriptions are given in Table 5 and total amounts of formed impurities after 6 months and the intragranular drug load are given in Table 6. The results show that high intragranular drug load improves stability in wet granulated compositions.

**Table 5. Quantitative formulation of wet granulated (WG) compositions**

| **Composition (%)** | | | | | |
|---|---|---|---|---|---|
| | **WG11** | **WG12** | **WG13** | **WG14*** | **WG15** |
| **Intragranular** | | | | | |
| Compound (I), toluenesulfonic acid salt | 38.40 | 14.12 | 8.31 | 4.71 | 1.88 |
| Mannitol | 36.30 | 57.72 | 54.95 | 57.94 | 60.32 |
| Pregelatinized starch | 15.50 | 19.24 | 12.27 | 12.88 | 13.33 |
| HPMC | 3.80 | 3.92 | 3.77 | 3.77 | 3.77 |
| Croscarmellose sodium | 1.90 | 2.00 | 1.88 | 1.88 | 1.88 |

| **Extragranular** | | | | | |
|---|---|---|---|---|---|
| Microcrystalline cellulose | - | - | 15.06 | 15.06 | 15.06 |
| Croscarmellose sodium | 1.00 | 1.00 | 1.88 | 1.88 | 1.88 |
| Sodium stearyl fumarate | 2.00 | 2.00 | 1.88 | 1.88 | 1.88 |
| Total % | **100 %** | **100 %** | **100 %** | **100 %** | **100** % |
| Total tablet weight | 90.9 mg | 50 mg | 85 mg | 75 mg | 75 mg |

| | | | | | |
|---|---|---|---|---|---|
| * Contains also a coating (HPMC, TiO₂, talc, polydextrose and PEG 3350) amounting 4 % of core tablet weight | | | | | |

**Table 6. Results of the comparative stability study**

| **Composition** | **Intragranular drug load** | **Total impurities** |
|---|---|---|
| WG11 | 40.0 % | < LOQ |
| WG12 | 14.6 % | 0.1 % |
| WG13 | 10.2 % | 0.5 % |
| WG14 | 5.8 % | 0.7 % |
| WG15 | 2.3 % | 1.9 % |

| | | |
|---|---|---|
| LOQ = limit of quantitation | | |

### Example 18. Comparative stability study IV

The effect of oxygen scavenger on the stability of the tablets WG 14 and WG 15 (see Example 17) was studied. The stability of the tablets was tested by storing 65 tablets in closed 60 ml HDPE bottle with 2 g of silica desiccant in 40 °C / 75 % RH conditions for 6 months. The stability was tested with or without oxygen scavenger canisters (PharmaKeep^{®} Capsule CD-2.15G, Airnov Healthcare Packaging for WG14 and StabilOx^{®}, Multisorb Filtration Group for WG 15) inside the bottle. The results are shown in Table 7. The results show that inclusion of an oxygen scavenger improved stability of the composition.

**Table 7. Results of the comparative stability study**

| **Composition** | **Description** | **Total impurities** |
|---|---|---|
| WG14 | Without oxygen scavenger | 0.7 % |
| WG14 | With oxygen scavenger | 0.1 % |
| WG15 | Without oxygen scavenger | 1.9 % |
| WG15 | With oxygen scavenger | 1.0 % |

## Claims

1. A wet granulated pharmaceutical composition comprising 2-(isoindolin-2-ylmethyl)-5-((1-(methylsulfonyl)piperidin-4-yl)methoxy)-4H-pyran-4-one (I) or a pharmaceutically acceptable salt thereof and at least one excipient.

2. The pharmaceutical composition according to claim 1, comprising an intragranular part which comprises at least about 5 %, per weight of the intragranular part, of 2-(isoindolin-2-ylmethyl)-5-((1-(methylsulfonyl)piperidin-4-yl)methoxy)-4H-pyran-4-one (I) or a pharmaceutically acceptable salt thereof, and at least one excipient.

3. The pharmaceutical composition according to claim 2, wherein the intragranular part comprises from about 5 % to about 20 %, per weight of the intragranular part, of 2-(isoindolin-2-ylmethyl)-5-((1-(methylsulfonyl)piperidin-4-yl)methoxy)-4H-pyran-4-one (I) or a pharmaceutically acceptable salt thereof.

4. The pharmaceutical composition according to any one of claims 1 to 3 comprising:
(a) an intragranular part comprising at least 5 %, per weight of the intragranular part, of 2-(isoindolin-2-ylmethyl)-5-((1-(methylsulfonyl)piperidin-4-yl)methoxy)-4H-pyran-4-one (I) or a pharmaceutically acceptable salt thereof, and at least one excipient; and
(b) an extragranular part comprising at least one excipient.

5. The pharmaceutical composition according to any one of claims 1 to 4, comprising from about 0.5 mg to about 10 mg of 2-(isoindolin-2-ylmethyl)-5-((1-(methylsulfonyl)-piperidin-4-yl)methoxy)-4H-pyran-4-one (I) or a pharmaceutically acceptable salt thereof.

6. The pharmaceutical composition according to any one of claims 1 to 5, comprising:
(a) an intragranular part comprising, per weight of the intragranular part,
(i) from about 5 % to about 20 %, of 2-(isoindolin-2-ylmethyl)-5-((1-(methylsulfonyl)piperidin-4-yl)methoxy)-4H-pyran-4-one (I) or a pharmaceutically acceptable salt thereof;
(ii) from about 55 % to about 95 % of a filler;
(iii) from about 0.5 % to about 10 % of a disintegrant; and
(iv) from about 1 % to about 15 % of a binder; and
(b) an extragranular part comprising, per weight of the extragranular part,
(i) from about 50 % to 100 % of a filler;
(ii) from 0 % to about 25 % of a disintegrant; and
(iii) from 0 % to about 25 % of a lubricant.

7. The pharmaceutical composition according to claim 6, wherein:
(i) the filler is selected from mannitol, pregelatinized starch, microcrystalline cellulose, lactose and a calcium hydrogen phosphate; and/or
(ii) the disintegrant comprises croscarmellose sodium or sodium starch glycolate; and/or
(iii) the binder comprises HPMC or HPC; and/or
(iv) the lubricant comprises sodium stearyl fumarate;
optionally wherein the binder comprises HPMC and the disintegrant comprises croscarmellose sodium.

8. The pharmaceutical composition according to any one of claims 1 to 7, comprising:
(a) an intragranular part comprising, per weight of the intragranular part,
(i) from about 5 % to about 20 %, of 2-(isoindolin-2-ylmethyl)-5-((1-(methylsulfonyl)piperidin-4-yl)methoxy)-4H-pyran-4-one (I) or a pharmaceutically acceptable salt thereof;
(ii) from about 55 % to about 95 % of a filler selected from mannitol, pregelatinized starch, microcrystalline cellulose, lactose, calcium hydrogen phosphate and a combination thereof;
(iii) from about 0.5 % to about 10 % of a disintegrant selected from croscarmellose sodium, sodium starch glycolate and a combination thereof; and
(iv) from about 1 % to about 15 % of binder comprising HPMC, HPC or combination thereof; and
(b) an extragranular part comprising, per weight of the extragranular part,
(i) from about 50 % to 100 % of a filler selected from microcrystalline cellulose, mannitol, lactose, calcium hydrogen phosphate and a combination thereof;
(ii) from 0 % to about 25 % of a disintegrant selected from croscarmellose sodium, sodium starch glycolate and a combination thereof; and
(iii) from 0 % to about 25 % of a lubricant comprising sodium stearyl fumarate.

9. The pharmaceutical composition according to claim 8, comprising:
(a) an intragranular part comprising, per weight of the intragranular part,
(i) from about 5 % to about 20 %, of 2-(isoindolin-2-ylmethyl)-5-((1-(methylsulfonyl)piperidin-4-yl)methoxy)-4H-pyran-4-one (I) or a pharmaceutically acceptable salt thereof;
(ii) from about 55 % to about 95 % of a combination of mannitol and pregelatinized starch;
(iii) from about 0.5 % to about 10 % of croscarmellose sodium
(iv) from about 1 % to about 15 % of HPMC; and
(b) an extragranular part comprising, per weight of the extragranular part,
(i) from about 50 % to 100 % of microcrystalline cellulose;
(ii) from 0 % to about 25 % of croscarmellose sodium; and
(iii) from 0 % to about 25 % of sodium stearyl fumarate.

10. The pharmaceutical composition according to any one of claims 2 to 9, wherein the intragranular part comprises, from about 30 % to about 95 %, from about 50 % to about 90 %, or from about 70 % to about 85 %, per weight of the composition.

11. The pharmaceutical composition according to any one of claims 1 to 10, which is in the form of a tablet, or a powder suitable to be filled into a capsule shell.

12. The pharmaceutical composition according to any one of claims 1 to 11, comprising*p-*toluenesulfonic acid salt of 2-(isoindolin-2-ylmethyl)-5-((1-(methylsulfonyl)piperidin-4-yl)methoxy)-4H-pyran-4-one (I).

13. The pharmaceutical composition according to any one of claims 1 to 12, wherein the composition provides a dissolution of at least 80 % per weight of 2-(isoindolin-2-yl-methyl)-5-((1-(methylsulfonyl)piperidin-4-yl)methoxy)-4H-pyran-4-one (I) or a pharmaceutically acceptable salt thereof within 60 minutes, or within 45 minutes, in 0.05 M phosphate buffer pH 6.8 using paddle apparatus (USP apparatus 2) with paddle speed of 75 rpm at 37 °C and vessel volume of 500 ml.

14. The pharmaceutical composition according to any one of claims 1 to 13, wherein the volume median diameter (Dv50) of particles of 2-(isoindolin-2-ylmethyl)-5-((1-(methylsulfonyl)piperidin-4-yl)methoxy)-4H-pyran-4-one (I) or a pharmaceutically acceptable salt thereof is not more than 50 µm, not more than 30 µm, or not more than 25 µm.

15. A package comprising a wet granulated pharmaceutical composition according to any one of claims 1 to 14, together with an oxygen scavenger.

## Patentansprüche

1. Feuchtgranulierte pharmazeutische Zusammensetzung, umfassend 2-(Isoindolin-2-ylmethyl)-5-((1-(methylsulfonyl)piperidin-4-yl)methoxy)-4H-pyran-4-on (I) oder ein pharmazeutisch verträgliches Salz davon und mindestens einen Hilfsstoff.

2. Pharmazeutische Zusammensetzung gemäß Anspruch 1, umfassend einen intragranulären Teil, welcher mindestens etwa 5 %, bezogen auf das Gewicht des intragranulären Teils, von 2-(Isoindolin-2-ylmethyl)-5-((1-(methylsulfonyl)piperidin-4-yl)methoxy)-4H-pyran-4-on (I) oder einem pharmazeutisch verträglichen Salz davon und mindestens einen Hilfsstoff umfasst.

3. Pharmazeutische Zusammensetzung gemäß Anspruch 2, wobei der intragranuläre Teil etwa 5 % bis etwa 20 %, bezogen auf das Gewicht des intragranulären Teils, von 2- (Isoindolin-2-ylmethyl)-5-((1-(methylsulfonyl)piperidin-4-yl)methoxy)-4H-pyran-4-on (I) oder einem pharmazeutisch verträglichen Salz davon umfasst.

4. Pharmazeutische Zusammensetzung gemäß einem der Ansprüche 1 bis 3, umfassend:
(a) einen intragranulären Teil, welcher mindestens 5 %, bezogen auf das Gewicht des intragranulären Teils, von 2-(Isoindolin-2-ylmethyl)-5-((1-(methylsulfonyl)piperidin-4-yl)methoxy)-4H-pyran-4-on (I) oder einem pharmazeutisch verträglichen Salz davon und mindestens einen Hilfsstoff umfasst; und
(b) einen extragranulären Teil, welcher mindestens einen Hilfsstoff umfasst.

5. Pharmazeutische Zusammensetzung gemäß einem der Ansprüche 1 bis 4, umfassend etwa 0,5 mg bis etwa 10 mg von 2-(Isoindolin-2-ylmethyl)-5-((1-(methylsulfonyl)-piperidin-4-yl)methoxy)-4H-pyran-4-on (I) oder einem pharmazeutisch verträglichen Salz davon.

6. Pharmazeutische Zusammensetzung gemäß einem der Ansprüche 1 bis 5, umfassend:
(a) einen intragranulären Teil, umfassend, bezogen auf das Gewicht des intragranulären Teils,
(i) etwa 5 % bis etwa 20 % von 2-(Isoindolin-2-ylmethyl)-5-((1-(methyl-sulfonyl)piperidin-4-yl)methoxy)-4H-pyran-4-on (I) oder einem pharmazeutisch verträglichen Salz davon;
(ii) etwa 55 % bis etwa 95 % eines Füllstoffs;
(iii) etwa 0,5 % bis etwa 10 % eines Desintegrationsmittels; und
(iv) etwa 1 % bis etwa 15 % eines Bindemittels; und
(b) einen extragranulären Teil, umfassend, bezogen auf das Gewicht des extragranulären Teils,
(i) etwa 50 % bis etwa 100 % eines Füllstoffs;
(ii) etwa 0 % bis etwa 25 % eines Desintegrationsmittels; und
(iii) etwa 0 % bis etwa 25 % eines Gleitmittels.

7. Pharmazeutische Zusammensetzung gemäß Anspruch 6, wobei:
(i) der Füllstoff ausgewählt ist aus Mannitol, vorgelatinierter Stärke, mikrokristalliner Cellulose, Lactose und einem Calciumhydrogenphosphat; und/oder
(ii) das Desintegrationsmittel Natriumcroscarmellose oder Natriumstärkeglycolat umfasst; und/oder
(iii) das Bindemittel HPMC oder HPC umfasst; und/oder
(iv) das Gleitmittel Natriumstearylfumarat umfasst;
wobei optional das Bindemittel HPMC umfasst und das Desintegrationsmittel Natriumcroscarmellose umfasst.

8. Pharmazeutische Zusammensetzung gemäß einem der Ansprüche 1 bis 7, umfassend:
(a) einen intragranulären Teil, umfassend, bezogen auf das Gewicht des intragranulären Teils,
(i) etwa 5 % bis etwa 20 % von 2-(Isoindolin-2-ylmethyl)-5-((1-(methylsulfonyl)piperidin-4-yl)methoxy)-4H-pyran-4-on (I) oder einem pharmazeutisch verträglichen Salz davon;
(ii) etwa 55 % bis etwa 95 % eines Füllstoffs, ausgewählt aus Mannitol, vorgelatinierter Stärke, mikrokristalliner Cellulose, Lactose, Calciumhydrogenphosphat und einer Kombination davon;
(iii) etwa 0,5 % bis etwa 10 % eines Desintegrationsmittels, ausgewählt aus Natriumcroscarmellose, Natriumstärkeglycolat und einer Kombination davon; und
(iv) etwa 1 % bis etwa 15 % eines Bindemittels, umfassend HPMC, HPC oder eine Kombination davon; und
(b) einen extragranulären Teil, umfassend, bezogen auf das Gewicht des extragranulären Teils,
(i) etwa 50 % bis 100 % eines Füllstoffs, ausgewählt aus mikrokristalliner Cellulose, Mannitol, Lactose, Calciumhydrogenphosphat und einer Kombination davon;
(ii) 0 % bis etwa 25 % eines Desintegrationsmittels, ausgewählt aus Natriumcroscarmellose, Natriumstärkeglycolat und einer Kombination davon; und
(iii) 0 % bis etwa 25 % eines Gleitmittels, umfassend Natriumstearylfumarat.

9. Pharmazeutische Zusammensetzung gemäß Anspruch 8, umfassend:
(a) einen intragranulären Teil, umfassend, bezogen auf das Gewicht des intragranulären Teils,
(i) etwa 5 % bis etwa 20 % von 2-(Isoindolin-2-ylmethyl)-5-((1-(methylsulfonyl)piperidin-4-yl)methoxy)-4H-pyran-4-on (I) oder einem pharmazeutisch verträglichen Salz davon;
(ii) etwa 55 % bis etwa 95 % einer Kombination aus Mannitol und vorgelatinierter Stärke;
(iii) etwa 0,5 % bis etwa 10 % von Natriumcroscarmellose;
(iv) etwa 1 % bis etwa 15 % von HPMC; und
(b) einen extragranulären Teil, umfassend, bezogen auf das Gewicht des extragranulären Teils,
(i) etwa 50 % bis 100 % von mikrokristalliner Cellulose;
(ii) etwa 0 % bis etwa 25 % von Natriumcroscarmellose; und
(iii) 0 % bis etwa 25 % von Natriumstearylfumarat.

10. Pharmazeutische Zusammensetzung gemäß einem der Ansprüche 2 bis 9, wobei der intragranuläre Teil etwa 30 % bis etwa 95 %, etwa 50 % bis etwa 90 % oder etwa 70 % bis etwa 85 % umfasst, bezogen auf das Gewicht der Zusammensetzung.

11. Pharmazeutische Zusammensetzung gemäß einem der Ansprüche 1 bis 10, welche in Form einer Tablette oder eines zum Einfüllen in eine Kapselhülle geeigneten Pulvers vorliegt.

12. Pharmazeutische Zusammensetzung gemäß einem der Ansprüche 1 bis 11, umfassend das p-Toluolsulfonsäuresalz von 2-(Isoindolin-2-ylmethyl)-5-((1-(methylsulfonyl)piperidin-4-yl)methoxy)-4H-pyran-4-on (I).

13. Pharmazeutische Zusammensetzung gemäß einem der Ansprüche 1 bis 12, wobei die Zusammensetzung eine Lösung von mindestens 80 %, bezogen auf das Gewicht von 2-(Isoindolin-2-yl-methyl)-5-((1-(methylsulfonyl)piperidin-4-yl)methoxy)-4H-pyran-4-on (I) oder eines pharmazeutisch verträglichen Salzes davon, innerhalb von 60 Minuten oder innerhalb von 45 Minuten bereitstellt, in 0,05 M Phosphatpuffer mit einem pH-Wert von 6,8 unter Verwendung einer Paddelvorrichtung (USP-Vorrichtung 2) mit einer Paddeldrehzahl von 75 U/min bei 37 °C und einem Gefäßvolumen von 500 ml.

14. Pharmazeutische Zusammensetzung gemäß einem der Ansprüche 1 bis 13, wobei der Volumenmediandurchmesser (Dv50) der Partikel von 2-(Isoindolin-2-ylmethyl)-5-((1-(methylsulfonyl)piperidin-4-yl)methoxy)-4H-pyran-4-on (I) oder einem pharmazeutisch verträglichen Salz davon nicht mehr als 50 µm, nicht mehr als 30 µm oder nicht mehr als 25 µm beträgt.

15. Packung, umfassend eine feuchtgranulierte pharmazeutische Zusammensetzung nach einem der Ansprüche 1 bis 14, zusammen mit einem Sauerstofffänger.

## Revendications

1. Une composition pharmaceutique granulaire humide comprenant de la 2-(isoindolin-2-ylméthyl)-5-((1-(méthylsulfonyl)pipéridin-4-yl)méthoxy)-4H-pyran-4-one (I) ou un sel pharmaceutiquement acceptable de celle-ci et au moins un excipient.

2. La composition pharmaceutique selon la revendication 1, comprenant une partie intragranulaire qui comprend au moins environ 5 %, par rapport à la partie intragranulaire, de 2-(iso-indolin-2-ylméthyl)-5-((1-(méthylsulfonyl)pipéridin-4-yl)méthoxy)-4H-pyran-4-one (I) ou un sel pharmaceutiquement acceptable de celle-ci, et au moins un excipient.

3. La composition pharmaceutique selon la revendication 2, dans laquelle la partie intragranulaire comprend d'environ 5 % à environ 20 %, par rapport à la partie intragranulaire, de 2-(isoindolin-2-ylméthyl)-5-((1-(méthylsulfonyl)pipéridin-4-yl)méthoxy)-4H-pyran-4-one (I) ou un sel pharmaceutiquement acceptable de celle-ci.

4. La composition pharmaceutique selon l'une quelconque des revendications 1 à 3 comprenant :
(a) une partie intragranulaire comprenant au moins 5 %, par rapport à la partie intragranulaire, de 2-(isoindolin-2-ylméthyl)-5-((1-(méthylsulfonyl)pipéridin-4-yl)méthoxy)-4H-pyran-4-one (I) ou un sel pharmaceutiquement acceptable de celle-ci, et au moins un excipient ; et
(b) une partie extragranulaire comprenant au moins un excipient.

5. La composition pharmaceutique selon l'une quelconque des revendications 1 à 4, comprenant d'environ 0,5 mg à environ 10 mg de 2-(isoindolin-2-ylméthyl)-5-((1-(méthylsulfonyl)-pipéridin-4-yl)méthoxy)-4H-pyran-4-one (I) ou un sel pharmaceutiquement acceptable de celle-ci.

6. La composition pharmaceutique selon l'une quelconque des revendications 1 à 5, comprenant :
(a) une partie intragranulaire comprenant, par rapport à la partie intragranulaire,
(i) d'environ 5 % à environ 20 % de 2-(isoindolin-2-ylméthyl)-5-((1-(méthyl-sulfonyl)pipéridin-4-yl)méthoxy)-4H-pyran-4-one (I) ou un sel pharmaceutiquement acceptable de celle-ci ;
(ii) d'environ 55 % à environ 95 % d'une charge ;
(iii) d'environ 0,5 % à environ 10 % d'un désintégrant ; et
(iv) d'environ 1 % à environ 15 % d'un liant ; et
(b) une partie extragranulaire comprenant, par rapport à la partie extragranulaire,
(i) d'environ 50 % à 100 % d'une charge ;
(ii) de 0 % à environ 25 % d'un désintégrant ; et
(iii) de 0 % à environ 25 % d'un lubrifiant.

7. La composition pharmaceutique selon la revendication 6, dans laquelle :
(i) la charge est choisie parmi le mannitol, l'amidon prégélatinisé, la cellulose microcristalline, le lactose et un hydrogène phosphate de calcium ; et/ou
(ii) le désintégrant comprend du croscarmellose sodique ou du glycolate d'amidon sodique ; et/ou
(iii) le liant comprend HPMC ou HPC ; et/ou
(iv) le lubrifiant comprend du fumarate de stéaryle de sodium ;facultativement dans laquelle le liant comprend HPMC et le désintégrant comprend du croscarmellose sodique.

8. La composition pharmaceutique selon l'une quelconque des revendications 1 à 7, comprenant :
(a) une partie intragranulaire comprenant, par rapport à la partie intragranulaire,
(i) d'environ 5 % à environ 20 % de 2-(isoindolin-2-ylméthyl)-5-((1-(méthyl-sulfonyl)pipéridin-4-yl)méthoxy)-4H-pyran-4-one (I) ou un sel pharmaceutiquement acceptable de celle-ci ;
(ii) d'environ 55 % à environ 95 % d'une charge choisie parmi le mannitol, l'amidon prégélatinisé, la cellulose microcristalline, le lactose, l'hydrogène phosphate de calcium et une combinaison de ceux-ci ;
(iii) d'environ 0,5 % à environ 10 % d'un désintégrant choisi parmi le croscarmellose sodique, le glycolate d'amidon sodique et une combinaison de ceux-ci ; et
(iv) d'environ 1 % à environ 15 % de liant comprenant HPMC, HPC ou une combinaison de ceux-ci ; et
(b) une partie extragranulaire comprenant, par rapport à la partie extragranulaire,
(i) d'environ 50 % à 100 % d'une charge choisie parmi la cellulose microcristalline, le mannitol, le lactose, l'hydrogène phosphate de calcium et une combinaison de ceux-ci ;
(ii) de 0 % à environ 25 % d'un désintégrant choisi parmi le croscarmellose sodique, le glycolate d'amidon sodique et une combinaison de ceux-ci ; et
(iii) de 0 % à environ 25 % d'un lubrifiant comprenant du fumarate de stéaryle de sodium.

9. La composition pharmaceutique selon la revendication 8, comprenant :
(a) une partie intragranulaire comprenant, par rapport à la partie intragranulaire,
(i) d'environ 5 % à environ 20 % de 2-(isoindolin-2-ylméthyl)-5-((1-(méthyl-sulfonyl)pipéridin-4-yl)méthoxy)-4H-pyran-4-one (I) ou un sel pharmaceutiquement acceptable de celle-ci ;
(ii) d'environ 55 % à environ 95 % d'une combinaison de mannitol et d'amidon prégélatinisé ;
(iii) d'environ 0,5 % à environ 10 % de croscarmellose sodique
(iv) d'environ 1 % à environ 15 % de HPMC ; et
(b) une partie extragranulaire comprenant, par rapport à la partie extragranulaire,
(i) d'environ 50 % à 100 % de cellulose microcristalline ;
(ii) de 0 % à environ 25 % de croscarmellose sodique ; et
(iii) de 0 % à environ 25 % de fumarate de stéaryle de sodium.

10. La composition pharmaceutique selon l'une quelconque des revendications 2 à 9, dans laquelle la partie intragranulaire comprend d'environ 30 % à environ 95 %, d'environ 50 % à environ 90 %, ou d'environ 70 % à environ 85 %, par rapport à la composition.

11. La composition pharmaceutique selon l'une quelconque des revendications 1 à 10, qui est dans la forme d'un comprimé, ou d'une poudre adaptée à être versée dans une enveloppe de capsule.

12. La composition pharmaceutique selon l'une quelconque des revendications 1 à 11, comprenant un sel d'acide *p*-toluènesulfonique de 2-(isoindolin-2-ylméthyl)-5-((1-(méthylsulfonyl)pipéridin-4-yl)méthoxy)-4H-pyran-4-one (I).

13. La composition pharmaceutique selon l'une quelconque des revendications 1 à 12, dans laquelle la composition fournit une dissolution d'au moins 80 % par rapport au 2-(isoindolin-2-yl-méthyl)-5-((1-(méthylsulfonyl)pipéridin-4-yl)méthoxy)-4H-pyran-4-one (I) ou un sel pharmaceutiquement acceptable de celle-ci dans un délai de 60 minutes, ou dans un délai de 45 minutes, dans un tampon phosphate 0,05 M pH 6,8 en utilisant un appareil à palettes (l'appareil USP 2) avec une vitesse de palettes de 75 tr/min à 37 °C et un volume de récipient de 500 ml.

14. La composition pharmaceutique selon l'une quelconque des revendications 1 à 13, dans laquelle le diamètre médian en volume (Dv50) de particules de 2-(isoindolin-2-ylméthyl)-5-((1-(méthylsulfonyl)pipéridin-4-yl)méthoxy)-4H-pyran-4-one (I) ou un sel pharmaceutiquement acceptable de celle-ci n'est pas supérieur à 50 µm, pas supérieur à 30 µm, ou pas supérieur à 25 µm.

15. Un emballage comprenant une composition pharmaceutique granulée par voie humide selon l'une quelconque des revendications 1 à 14, avec un absorbeur d'oxygène.
